Europäisches Patentamt

**European Patent Office**

**Office européen des brevets**

(19)

(11) Numéro de publication : **0 002 630**

**B2**

(12) **NOUVEAU FASCICULE DE BREVET EUROPÉEN**

(45) Date de publication du nouveau fascicule du brevet :
**16.04.86**

(21) Numéro de dépôt : **78400202.4**

(22) Date de dépôt : **01.12.78**

(51) Int. Cl.⁴ : **B 01 J 23/74**, C 07 C 85/06,
C 07 D295/00.

(54) Catalyseur et procédé de fabrication d'amines à partir d'alcools.

(30) Priorité : **12.12.77 FR 7737346**
**04.07.78 FR 7819875**

(43) Date de publication de la demande :
**27.06.79 Bulletin 79/13**

(45) Mention de la délivrance du brevet :
**10.06.81 Bulletin 81/23**

(45) Mention de la décision concernant l'opposition :
**16.04.86 Bulletin 86/16**

(84) Etats contractants désignés :
**BE CH DE FR GB IT LU NL SE**

(56) Documents cités :
**EP-A- 0 000 055**
**DE-A- 1 950 604**
**DE-A- 1 953 263**
**DE-A- 2 024 282**
**DE-A- 2 445 303**
**FR-A- 2 310 802**
**FR-A- 2 325 427**
**FR-A- 2 337 585**
**US-A- 3 151 115**
**Le dossier contient des informations techniques présentées postérieurement au dépôt de la demande et ne figurant pas dans le présent fascicule.**

(73) Titulaire : **SOCIETE CHIMIQUE DE LA GRANDE PAROISSE AZOTE ET PRODUITS CHIMIQUES**
**8, rue Cognacq-Jay**
**F-75321 Paris Cedex 07 (FR)**

(72) Inventeur : **Le Goff, Yannick**
**99, avenue de Plaisance**
**F-44600 Saint Nazaire (FR)**
Inventeur : **Senes, Michel**
**11, avenue des Flandres**
**F-44500 La Baule (FR)**
Inventeur : **Hamon, Christian**
**47, Chemin des Dames**
**F-44600 Saint Nazaire (FR)**

(74) Mandataire : **Bouton Neuvy, Liliane et al**
**L'Air liquide, Société Anonyme pour L'Etude et L'Exploitation des Procédés Georges Claude 75, Quai d'Orsay**
**F-75321 Paris Cedex 07 (FR)**

Jouve, 18, rue St-Denis, 75001 Paris, France

## Description

La présente invention concerne un catalyseur pour la mise en œuvre d'un procédé de fabrication d'amines à partir d'alcools en particulier d'éthanolamine.

Il existe principalement deux voies de fabrication industrielle de l'éthylène diamine. La plus grande partie des capacités de production est basée sur le dichloroéthane ; cependant ce procédé conduit à la production de chlorure de sodium pollué par des amines qui rendent sa récupération difficile. Aussi, la fabrication d'amines à partir d'éthanolamine représente-t-elle une voie intéressante à condition de conduire la réaction de l'éthanolamine et de l'ammoniac en présence d'un catalyseur efficace, sélectif et ayant une longue durée de vie.

On a proposé des catalyseurs à base d'oxyde de magnésium, mais il s'avère que ces catalyseurs se dégradent au contact du milieu réactionnel ; l'hydratation du support magnésique provoquant un affaiblissement de la résistance mécanique et une décroissance rapide de l'activité.

Dans le domaine de l'hydrogénation directe on connait par le brevet européen EP. A 0000 55, appartenant à l'état de la technique visé à l'article 54 (3) des catalyseurs d'hydrogénation contenant du cuivre, du nickel et (ou) du cobalt sur un support de silice obtenus par mélange de solutions aqueuses de sels de cuivre, de nickel et ou de cobalt et des solutions de silicates de métaux alcalins avec un rapport Me$_2$O : SiO$_2$ supérieur à 1 en des proportions telles que leur mélange possède un pH de 6 à 8 et la teneur en cuivre, nickel et (ou) cobalt du catalyseur terminé se situant entre 40 et 80 % en poids, les précipités formés au moment du mélange étant séparés, façonnés et séchés de manière usuelle et traités avec des agents réducteurs.

Par la demande de brevet France 2 337 585 on connait des catalyseurs au nickel/rhénium à une teneur de 3 à 30 % en poids du support formé notamment d'$\alpha$ - alumine, et dont la surface spécifique est égale ou supérieure à 1 m$^2$/g. En raison de la présence d'un métal précieux rare et d'un coût élevé, ces catalyseurs paraissent difficilement exploitables à l'échelle industrielle.

Il a été trouvé selon l'invention un catalyseur d'amination moins onéreux, contenant des métaux actifs facilement disponibles, plus performant et sélectif vis-à-vis de l'éthylène diamine que les catalyseurs précédents. L'efficacité de ce catalyseur est due à un choix rigoureux de sa porosité et à celui d'une teneur élevée en métal actif. Il catalyse notamment la réaction de l'éthanolamine et de l'ammoniac en vue de la production d'éthylènediamine, de pipérazine et de sous produits intéressants tels diéthylènetriamine, aminoéthylpipérazine, aminoéthyléthanolamine et hydroxyéthylpipérazine. Ce catalyseur a une excellente résistance mécanique qui est conservée, et une durée de vie supérieure à celle des catalyseurs connus pour cette application.

L'activité du catalyseur après une période six mois de marche est tout à fait semblable et plus particulièrement le rendement en éthylènediamine ne subit aucune variation par rapport aux résultats initiaux. L'activité du catalyseur reste parfaitement stable et il ne subit aucune altération mécanique après plus de 5 000 heures de marche. Les résultats de divers fonctionnements en continu confirment ces avantages.

En fonction du catalyseur et des différents paramètres du procédé, on peut orienter la réaction sélectivement vis à vis de l'éthylène diamine, de la pipérazine ou d'amines plus lourdes, ce qui permet d'adapter la fabrication à l'évolution du marché. Le catalyseur peut être avantageusement utilisé dans diverses réactions d'hydrogénation.

Le catalyseur d'amination est constitué par un élément actif de la famille des métaux de transition uniformément allié à un édifice poreux réfractaire de surface spécifique comprise entre 10 et 300 m$^2$/g et de diamètre de pores inférieur à 5 000 Å. Les catalyseurs dont la surface spécifique est comprise entre 30 et 50 m$^2$/g sont particulièrement avantageux.

Le métal actif préféré est le nickel, utilisé seul ou en association avec d'autres métaux de transition tels cuivre et cobalt ; le nickel représentant au moins 50 % de la matière active.

Le teneur en métaux de transition représente 30 à 70 % du poids total du catalyseur.

D'autre part, il a été découvert que la stabilité des catalyseurs est améliorée par la présence de sodium. On obtient des résultats avantageux en associant au métal actif un composé à base de sodium à une teneur comprise entre 0,15 et 20 % exprimée en sodium par rapport au poids du catalyseur. En particulier, une teneur de 5 à 10 % en sodium est choisie pour l'effet stabilisant qu'elle procure.

De plus, il a été remarqué que l'activité du catalyseur est exaltée par la présence d'un promoteur. Ce promoteur est de préférence sous forme d'un composé à base de rhodium à une teneur maximale de 0,1 % exprimée en poids de rhodium par rapport au poids du catalyseur.

Le support microporeux est au moins un oxyde choisi parmi le groupe constitué par l'alumine, la silice, l'oxyde de thorium et l'oxyde de cérium.

L'association du métal de transition et du support microporeux est soumise à une réduction directe sous balayage de gaz réducteur (tel mélange N$_2$/H$_2$) entre 400 et 550 °C, pendant plusieurs heures, de l'ordre de 8 à 10 heures, avec une montée en température de l'ambiante à la température de réduction de 50 °C par heure. Après réduction l'élément de transition se trouve sous forme métallique et réparti uniformément sur le support.

On peut avantageusement soumettre le catalyseur à une décomposition préalable par traitement thermique à l'air à une température comprise entre 350 et 500 °C, pendant quelques heures, avec une montée progressive en température depuis l'ambiante de 50 °C par heure jusqu'à la température de décomposition.

L'édifice structural du catalyseur peut être obtenu par co-précipitation de tous les éléments du catalyseur à partir de sels solubles par une base ou un carbonate, ou à partir de sels solubles en milieu basique par abaissement du pH. L'édifice structural peut également être constitué par imprégnation d'un sel soluble des éléments actifs sur les oxydes réfractaires préalablement combinés ; l'imprégnation pouvant avantageusement être conduite sous vide.

La co-précipitation de tous les éléments du catalyseur est avantageusement mise en œuvre à partir de sels solubles, tels les nitrates.

L'agent précipitant, utilisé en quantité stoechiométrique, est généralement une base (ammoniaque, hydroxyde de sodium ou potassium) ou un carbonate. Le précipité obtenu constitué par un mélange d'hydroxydes est filtré, puis lavé et séché, à température comprise entre 80 et 120 °C.

La co-précipitation de tous les éléments peut également être obtenue à partir de sels solubles en milieu basique, par abaissement du pH. Par exemple, pour la préparation d'un catalyseur nickel sur alumine ou silice, le nickel se trouve sous forme de complexe $[Ni(NH_3)_4]^{2+}$ obtenu par addition d'un grand excès de $NH_4OH$ sur une solution de nitrate de nickel, l'aluminium étant sous forme d'aluminate de sodium et le silicium sous forme de silicate de sodium. Comme précédemment le précipité obtenu est filtré, lavé et séché à température comprise entre 80 et 120 °C.

L'élaboration de l'édifice structural par co-précipitation permet d'ajuster les métaux de transition à la teneur désirée.

Selon la variante par imprégnation on réalise séparément le support poreux constitué par un ou plusieurs des oxydes précédemment mentionnés. Le support est ensuite imprégné par un sel soluble de l'élément actif, puis soumis à un séchage à une température de l'ordre de 80 à 120 °C. Le sel soluble est le plus souvent un nitrate. On effectue avantageusement un traitement préalable du support par séchage ou dégazage et on met en œuvre l'imprégnation sous vide. On obtient ainsi une augmentation de la teneur en matière active déposée. Dans le même but l'imprégnation peut être réalisée en plusieurs stades avec séchage intermédiaire.

Le sodium sous forme de sels ou allié à l'édifice poreux réfractaire est introduit soit en cours de précipitation et dosé en cours d'élimination au lavage, soit par imprégnation sur le catalyseur à un stade quelconque de sa fabrication.

Le catalyseur d'amination selon l'invention est avantageusement applicable à un procédé catalytique, en phase hétérogène, de production d'éthylènediamine et de pipérazine à partir d'éthanolamine et d'ammoniac. Ce procédé peut être mis en œuvre indifféremment en exploitation discontinue ou continue c'est-à-dire en réacteur fermé agité ou en réacteur ouvert avec circulation continue du mélange réactionnel sur le catalyseur.

La réaction de l'éthanolamine et de l'ammoniac en présence du catalyseur d'amination est conduite à une température comprise entre 170° et 260 °C, sous une pression comprise entre 50 et 300 bars absolus. On introduit dans le réacteur l'éthanolamine, l'ammoniac et de l'hydrogène en quantités telles que le rapport molaire ammoniac/éthanolamine soit compris entre 5 et 40 et le débit d'hydrogène soit compris entre 5 et 200 Nl par mole d'éthanolamine.

La réaction peut éventuellement être conduite en présence d'eau, celle-ci étant introduite dans le mélange réactionnel jusqu'à concurrence de 25 % en poids par rapport à l'éthanolamine.

On peut avantageusement envisager le recyclage des réactifs n'ayant pas réagi ou en excès tels l'éthanolamine non convertie et l'ammoniac en excès. Le recyclage partiel ou total d'un des réactants, de la pipérazine avec l'éthanolamine peut aussi constituer une possibilité intéressante.

Il est donné ci-après des exemples de préparation des catalyseurs et d'application du procédé en présence de ces catalyseurs avec recyclage éventuel.

Dans les exemples suivants, le terme « lourds » désigne l'ensemble des composés : diéthylènetriamine, aminoéthylpipérazine, aminoéthyléthanolamine et hydroxyéthylpipérazine. Les abréviations E.D.A., PIP et E.A. désignent respectivement l'éthylènediamine, la pipérazine et l'éthanolamine.

Exemple 1

Catalyseur 1 : (Nickel sur alumine) 459,7 g de nitrate d'aluminium cristallisé $[Al(NO_3)_3, 9H_2O]$ mis en solution dans 1 350 cm³ d'eau sont ajoutés à 286,5 cm³ d'une solution de nitrate de nickel de densité 1,53 et contenant 14,26 % en poids de nickel. Cette solution est ajoutée sous agitation dans 2,3 l d'une solution contenant 232,5 g de soude. La suspension obtenue est portée à l'ébullition pendant 10 mm. Après refroidissement, elle est filtrée sur büchner et lavée avec 2 l d'eau à 20 °C afin d'éliminer le nitrate de sodium. Le précipité est alors séché à 100 °C puis mis en forme en pastilles cylindriques (5 × 5 mm). Celles-ci sont ensuite décomposées sous air à 400 °C en montant progressivement la température à raison de 50 °C par heure et en maintenant le palier à 400 °C pendant 6 h. Le catalyseur ainsi obtenu est ensuite réduit sous mélange de gaz réducteur $(N_2 + 3H_2)$ à 450 °C pendant 8 h ; la montée en température étant de 50 °C/h. La teneur en Ni du catalyseur est de 50 % et sa surface spécifique est de 116 m²/g.

Application : dans un autoclave agité, de 550 cm³, on charge 50 g d'éthanolamine, 140 g d'ammoniac, 7,46 g du catalyseur 1 et 11,8 NI d'hydrogène. Le rapport molaire NH$_3$/E.A. est de 10, la quantité d'hydrogène de 14,4 NI/mole E.A. On maintient en palier à 205 °C pendant 8 h sous agitation, la pression est de 198 bars absolus. L'analyse du produit réactionnel est effectuée par chromatographie phase gazeuse, les résultats sont regroupés dans les tableaux ci-après.

| Taux de transformation de E.A. | % |
|---|---|
| en E.D.A. | 30,6 |
| en PIP | 15,2 |
| en lourds | 10,5 |
| TOTAL | 56,3 |

| Rendements % pds/E.A. entrée | |
|---|---|
| en E.D.A. | 30,2 |
| en PIP | 10,7 |
| en lourds | 8,0 |
| en H$_2$O | 16,4 |
| en E.A. non transformée | 43,7 |
| TOTAL | 109,0 |

| Rendements % pds E.A. consommée | |
|---|---|
| en E.D.A. | 53,6 |
| en PIP | 19,0 |
| en lourds | 14,2 |

## Exemple 2

Catalyseur 2. (Nickel sur alumine). Dans 2,8 l d'eau sont mis en solution : 735,5 g de nitrate d'aluminium cristallisé Al(NO$_3$)$_3$, 9H$_2$O et 495,4 g de nitrate de nickel cristallisé Ni(NO$_3$)$_2$, 6H$_2$O. Cette solution est versée sous agitation dans 5 l d'une solution de potasse contenant 521 g de KOH. Le précipité est filtré puis lavé avec 6 l d'eau déminéralisée, il est séché à 90 °C pendant 12 h puis décomposé sous air à 400 °C pendant 5 h en montant la température à raison de 50 °C/h. Après pastillage (5 × 5 mm), il est réduit sous mélange N$_2$ + 3H$_2$ (400 NI/h) en montant la température de l'ambiante jusqu'à 450 °C à raison de 50 °C/h, le palier à 450 °C étant de 10 h. La teneur en Ni du catalyseur est de 50 %, celle en potassium résiduel de 2,4 % et sa surface spécifique est de 156 m$^2$/g.

Application. Dans le même autoclave que précédemment, on charge 50 g d'éthanolamine, 140 g d'ammoniac, 7,50 g du catalyseur 2 et 11,8 NI d'hydrogène. On maintient en palier à 205 °C pendant 8 h sous agitation, la pression est de 204 bars absolus. Les résultats sont regroupés dans les tableaux ci-après.

| Taux de transformation de E.A. | % |
|---|---|
| en E.D.A. | 46,2 |
| en PIP | 14,8 |
| en lourds | 9,2 |
| TOTAL | 70,2 |

| Rendements % pds/E.A. entrée | |
|---|---|
| en E.D.A. | 45,4 |
| en PIP | 10,4 |
| en lourds | 7,4 |
| en H$_2$O | 20,6 |
| en E.A. non transformée | 29,9 |
| TOTAL | 113,7 |

| Rendements % pds/E.A. consommée | |
|---|---|
| en E.D.A. | 64,8 |
| en PIP | 14,9 |
| en lourds | 10,5 |

## Exemple 3

Catalyseur 3. (Nickel sur alumine) 260 cm$^3$ d'ammoniaque concentrée à 13 moles/l sont ajoutés à une solution de 320 cm$^3$ de nitrate de nickel contenant 49,5 g de nitrate de nickel cristallisé Ni(NO$_3$)$_2$, 6H$_2$O. La solution obtenue est bleulimpide, ceci étant dû à la formation avec l'ammoniac du complexe soluble nickel-tétramine. A cette solution est ajouté 28 cm$^3$ d'une solution d'aluminate de sodium de densité 1,52 et contenant 360 g/l en Al$_2$O$_3$. L'ensemble est introduit dans un ballon de 2 l et l'ammoniac est évaporé par entraînement à la vapeur d'eau surchauffée jusqu'à neutralité du milieu. Le précipité obtenu est alors filtré, lavé avec 2 l d'eau, puis séché à 80 °C. Après décomposition à l'air à 400 °C, il est mis en forme de pastilles cylindriques (5 × 5 mm), puis réduit à 450 °C sous mélange de gaz réducteur (N$_2$ + 3H$_2$), pendant 8 h, la montée en température pour la décomposition et la réduction est de 50 °C/heure. La teneur en Ni du catalyseur est de 50 % et sa surface spécifique est de 131 m$^2$/g.

4

**0 002 630**

Application : Dans l'autoclave de l'exemple 1 on charge 50 g d'éthanolamine, 140 g d'ammoniac, 10 g d'eau, 8,58 g du catalyseur 3 et 11,8 Nl d'hydrogène. On maintient en palier à 195 °C pendant 8 h, sous agitation ; la pression est de 160 bars absolus. Les résultats sont consignés dans les tableaux ci-après.

| Taux de transformation de E.A. | % |
|---|---|
| en E.D.A. | 31,4 |
| en PIP | 13,8 |
| en lourds | 11,3 |
| TOTAL | 56,5 |

| Rendements % pds/E.A. entrée | |
|---|---|
| en E.D.A. | 30,9 |
| en PIP | 9,7 |
| en lourds | 8,5 |
| en $H_2O$ | 36,2 |
| en E.A. non transformée | 43,5 |
| TOTAL | 128,8 |

| Rendements % pds/E.A. consommée | |
|---|---|
| en E.D.A. | 54,7 |
| en PIP | 17,3 |
| en lourds | 15,0 |

## Exemple 4

Catalyseur 4 : (Nickel sur alumine). On met en solution dans 5 l d'eau : 1 470 g de nitrate d'aluminium cristallisé et 778 g de nitrate de nickel cristallisé. On ajoute sous agitation 5,5 l d'une solution de soude contenant 685 g de NaOH. On filtre le précipité obtenu et on lave avec 11 l d'eau, le précipité est séché à 80 °C pendant 12 h puis décomposé sous air à 250 °C en montant la température à raison de 50 °C/h et en maintenant en palier pendant 5 h ; il est ensuite pastillé (5 × 5 mm). On le réduit à 450 °C pendant 8 h sous mélange $N_2 + 3H_2$ en montant progressivement la température à partir de l'ambiante à raison de 50 °C/h. Le poids de produit obtenu est de 350 g. La teneur en Ni du catalyseur est de 44 % et sa surface spécifique est de 164 m²/g.

Application : Dans l'autoclave de l'exemple 1, on charge 50 g d'éthanolamine, 140 g d'ammoniac, 10 g d'eau, 7,26 g du catalyseur 4 et 11,8 Nl d'hydrogène. On maintient en palier à 195 °C pendant 8 h, sous agitation ; la pression est de 160 bars absolus. Les résultats sont regroupés dans les tableaux ci-après.

| Taux de transformation de E.A. | % |
|---|---|
| en E.D.A. | 33,1 |
| en PIP | 5,0 |
| en lourds | 11,7 |
| TOTAL | 49,8 |

| Rendements % pds/E.A. entrée | |
|---|---|
| en E.D.A. | 32,6 |
| en PIP | 3,6 |
| en lourds | 8,7 |
| en $H_2O$ | 34,5 |
| en E.A. non transformée | 50,3 |
| TOTAL | 129,7 |

| Rendements % pds/E.A. consommée | |
|---|---|
| en E.D.A. | 65,6 |
| en PIP | 7,1 |
| en lourds | 17,6 |

## Exemple 5

Catalyseur 5 : (Nickel sur alumine). On met en solution 576,6 g de nitrate de nickel cristallisé dans 1,25 l d'eau. On ajoute 2,95 l d'une solution d'ammoniaque à 13,5 moles/l et on agite jusqu'à mise en solution de la totalité de l'hydroxyde de nickel ; on ajoute à cette solution 450 cm³ d'une solution d'aluminate de sodium contenant 211,5 g d'aluminate à 49,8 % en poids de $Al_2O_3$. On agite et on chasse l'ammoniac en excès par chauffage à 70 °C jusqu'à neutralité des vapeurs dégagées. Ce précipité obtenu est filtré et lavé avec 6 l d'eau déminéralisée. Il est séché à 80 °C pendant 12 h, décomposé à 300 °C pendant 5 h, puis réduit sous mélange $N_2 + 3H_2$ (400 Nl/h) à 450 °C pendant 8 h ; la montée en température pour la décomposition et la réduction est de 50 °C/h. La teneur en Ni du catalyseur est de 52,5 % et sa surface spécifique est de 124 m²/g.

Application : Dans l'autoclave de l'exemple 1, on charge 50 g d'éthanolamine, 140 g d'ammoniac, 10

5

# 0 002 630

g d'eau, 7,63 g du catalyseur 5 et 11,8 Nl d'hydrogène. On maintient en palier à 195 °C pendant 8 h, sous agitation ; la pression est de 128 bars absolus. Les résultats sont consignés dans les tableaux ci-après.

| Taux de transformation de E.A. | % |
|---|---|
| en E.D.A. | 32,0 |
| en PIP | 9,3 |
| en lourds | 7,8 |
| TOTAL | 49,1 |

| Rendements % pds/E.A. entrée | |
|---|---|
| en E.D.A. | 31,6 |
| en PIP | 6,5 |
| en lourds | 6,0 |
| en $H_2O$ | 34,4 |
| en E.A. non transformée | 51,0 |
| TOTAL | 129,5 |

| Rendements % pds/E.A. consommée | |
|---|---|
| en E.D.A. | 64,4 |
| en PIP | 13,3 |
| en lourds | 12,3 |

## Exemple 6

Catalyseur 6 : (Ni-Cu sur alumine). Dans 1,8 l d'eau sont mis en solution : 441,3 g de nitrate d'aluminium cristallisé, 267,5 g de nitrate de nickel cristallisé et 22,8 g de nitrate de cuivre cristallisé. Cette solution est ajoutée, sous agitation, dans 1,6 l d'une solution de soude contenant 222 g en NaOH. Le précipité est lavé avec 4 l d'eau à 20 °C, puis séché à 90 °C pendant 12 h. Il est alors broyé (refus au tamis 200 μ < 0,5 %) puis mélangé avec 1 % de graphite et mis en forme de pastilles cylindriques (5 × 5 mm). Il est ensuite réduit sous mélange $N_2$ + 3 $H_2$ (300 Nl/h) à 450 °C pendant 8 h ; la montée en température depuis l'ambiante a lieu à raison de 50 °C/h. La teneur en Ni du catalyseur est de 45 %, celle en Cu de 5 % et sa surface spécifique est de 136 m²/g.

Application : Dans l'autoclave de l'exemple 1 on charge 50 g d'éthanolamine, 140 g d'ammoniac, 10 g d'eau, 5,74 g du catalyseur 6 et 11,8 Nl d'hydrogène. On maintient en palier à 195 °C pendant 8 h, sous agitation ; la pression est de 149 bars absolus. Les résultats sont regroupés dans les tableaux ci-après.

| Taux de transformation de E.A. | % |
|---|---|
| en E.D.A. | 34,2 |
| en PIP | 13,0 |
| en lourds | 10,0 |
| TOTAL | 57,2 |

| Rendements % pds/E.A. entrée | |
|---|---|
| en E.D.A. | 33,6 |
| en PIP | 9,2 |
| en lourds | 7,8 |
| en $H_2O$ | 36,8 |
| en E.A. non transformée | 42,8 |
| TOTAL | 130,2 |

| Rendements % pds/E.A. consommée | |
|---|---|
| en E.D.A. | 58,8 |
| en PIP | 16,0 |
| en lourds | 13,6 |

## Exemple 7

Catalyseur 7 : (Ni-Co sur alumine). Dans 1,8 l d'eau sont mis en solution : 148,2 g de nitrate de cobalt cristallisé, 148,6 g de nitrate de nickel cristallisé et 441 g de nitrate d'aluminium cristallisé. On ajoute à cette solution, sous agitation, une solution de soude (2,4 l) contenant 222 g de NaOH. La suspension obtenue est filtrée puis lavée avec 4 l d'eau à 20 °C. Le précipité est séché à 90 °C pendant 12 h puis décomposé sous air à 400 °C pendant 5 h, en montant progressivement la température à raison de 50 °C/h. Après mise en forme (pastilles cylindriques 5 × 5 mm) il est réduit sous mélange $N_2$ + 3 $H_2$ (300 Nl/h) à 450 °C pendant 8 h (montée en température de 50 °C/h). La teneur en Ni du catalyseur est de 25 %, celle en Co de 25 %, celle en Na de 0,2 % et la surface spécifique est de 124 m²/g.

Application : Dans l'autoclave précédent, on charge 50 g d'éthanolamine, 140 g d'ammoniac, 7,8 g du catalyseur 7 et 11,8 Nl d'hydrogène. On maintient en palier à 205 °C pendant 8 h, sous agitation ; la pression est de 204 bars absolus. Les résultats sont regroupés dans les tableaux ci-après.

# 0 002 630

| Taux de transformation de E.A. | % |
|---|---|
| en E.D.A. | 37,2 |
| en PIP | 19,5 |
| en lourds | 10,3 |
| TOTAL | 67,0 |

| Rendements % pds/E.A. entrée | |
|---|---|
| en E.D.A. | 36,6 |
| en PIP | 13,8 |
| en lourds | 7,7 |
| en $H_2O$ | 19,6 |
| en E.A. non transformée | 33,1 |
| TOTAL | 110,8 |

| Rendements % pds/E.A. consommée | |
|---|---|
| en E.D.A. | 54,8 |
| en PIP | 20,6 |
| en lourds | 11,5 |

## Exemple 8

Catalyseur 8 : (Ni sur thorine). Dans 3,5 l d'eau déminéralisée sont dissous : 495 g de nitrate de nickel cristallisé et 313,7 g de nitrate de thorium cristallisé $Th(NO_3)_4$, 4 $H_2O$. On ajoute sous agitation 1,7 l d'une solution de soude contenant 227 g de NaOH. Le précipité est filtré puis lavé avec 6 l d'eau. Il est ensuite séché à 80 °C pendant 15 h et décomposé sous air à 400 °C pendant 5 h (montée en température 50 °C/h). Après mise en forme (pastilles 5 × 5 mm) il est réduit à 450 °C sous mélange $N_2$ + 3 $H_2$ (500 Nl/h) pendant 8 h (montée en température 50 °C/h). Le poids de produit obtenu est de 245 g. La teneur en Ni du catalyseur est de 40 %. Sa surface spécifique est de 60 $m^2$/g.

Application : Dans l'autoclave, on charge 50 g d'éthanolamine, 140 g d'ammoniac, 10 g d'eau, 6,80 g du catalyseur 8 et 11,8 Nl d'hydrogène. On maintient en palier à 195 °C pendant 8 h, sous agitation ; la pression est de 150 bars absolus. Les résultats sont regroupés dans les tableaux ci-après.

| Taux de transformation de E.A. | % |
|---|---|
| en E.D.A. | 38,3 |
| en PIP | 20,6 |
| en lourds | 5,0 |
| TOTAL | 63,9 |

| Rendements % pds/E.A. entrée | |
|---|---|
| en E.D.A. | 37,7 |
| en PIP | 14,5 |
| en lourds | 4,0 |
| en $H_2O$ | 38,8 |
| en E.A. non transformée | 36,1 |
| TOTAL | 131,1 |

| Rendements % pds/E.A. consommée | |
|---|---|
| en E.D.A. | 59,0 |
| en PIP | 22,7 |
| en lourds | 6,2 |

## Exemple 9

Catalyseur 9 : (Ni sur oxyde de cérium). Dans 2,6 l d'eau sont dissous : 495,6 g de nitrate de nickel cristallisé, 378,5 g de nitrate de cérium cristallisé $Ce(NO_3)_3$, 6 $H_2O$. On ajoute sous agitation 2 l d'une solution de soude contenant 270 g de NaOH. Le précipité est porté à 80 °C pendant 15 mn puis filtré après refroidissement à 40 °C et lavé avec 7,5 l d'eau déminéralisé. Il est traité sous air à 250 °C pendant 10 h (montée en température 50 °C/h). Le produit est broyé finement puis réduit dans un four horizontal sous balayage $N_2$ + 3 $H_2$, à 400 °C pendant 10 h (montée en température 50 °C/h). La teneur en Ni du catalyseur est de 40 % et sa surface spécifique est de 50 $m^2$/g.

Application : Dans l'autoclave, on charge 50 g d'éhtanolamine, 140 g d'ammoniac, 10 g d'eau, 6,83 g du catalyseur 9 et 11,8 Ni d'hydrogène. On maintient en palier à 195 °C pendant 8 h, sous agitation ; la pression est de 161 bars absolus. Les résultats sont consignés dans les tableaux ci-après.

(Voir Tableaux p. 8)

| Taux de transformation de E.A. | % |
|---|---|
| en E.D.A. | 32,4 |
| en PIP | 11,3 |
| en lourds | 3,3 |
| TOTAL | 47,0 |

| Rendements % pds/E.A. entrée | |
|---|---|
| en E.D.A. | 31,9 |
| en PIP | 8,0 |
| en lourds | 2,6 |
| en $H_2O$ | 33,8 |
| en E.A. non transformée | 53,0 |
| TOTAL | 129,3 |

| Rendements % pds/E.A. consommée | |
|---|---|
| en E.D.A. | 67,8 |
| en PIP | 17,0 |
| en lourds | 5,5 |

## Exemple 10

Catalyseur 10 : (Ni sur silice). A 420,8 g d'une solution de nitrate de nickel de densité 1,53 et contenant 14,26 % en poids de Ni, on ajoute 1,5 l d'une solution d'ammoniac à 13,5 moles/l. Le nickel se trouve alors sous forme du complexe soluble nickel tétramine. On ajoute sous forme agitation 222 g d'une solution de silicate de sodium à 27 % en poids de $SiO_2$. Tout en maintenant l'agitation l'excès d'ammoniac est évaporé lentement à 70-80 °C. Le précipité obtenu est filtré puis lavé avec 4 l d'eau déminéralisée. après séchage à 80 °C pendant 12 h, il est broyé, mélangé avec 1 % de graphite, mis en forme (pastilles cylindriques 5 × 5 mm) puis réduit directement sous mélange $N_2$ + 3 $H_2$ (300 Nl/h) à 450 °C pendant 8 h — (montée en température 50 °C/h) — La teneur en Ni du catalyseur est de 50 % et sa surface spécifique est de 146 m²/g.

Application : Dans l'autoclave précédent, on charge 50 g d'éthanolamine, 140 g d'ammoniac, 7,8 g du catalyseur 10 et 11,8 Ni d'hydrogène. On maintient en palier à 205 °C pendant 8 h, sous agitation ; la pression est de 189 bars absolus. Les résultats sont regroupés dans les tableaux ci-après.

| Taux de transformation de E.A. | % |
|---|---|
| en E.D.A. | 22,2 |
| en PIP | 15,4 |
| en lourds | 9,0 |
| TOTAL | 46,6 |

| Rendements % pds/E.A. entrée | |
|---|---|
| en E.D.A. | 21,8 |
| en PIP | 10,9 |
| en lourds | 6,5 |
| en $H_2O$ | 13,6 |
| en E.A. non transformée | 53,4 |
| TOTAL | 106,2 |

| Rendements % pds/E.A. consommée | |
|---|---|
| en E.D.A. | 46,9 |
| en PIP | 23,3 |
| en lourds | 13,9 |

## Exemple 11

Catalyseur 11 : (Ni sur silice-alumine) : 143 g de solution d'aluminate de sodium de densité 1,52, contenant 360 g/l en $Al_2O_3$ sont mélangés sous forte agitation avec 185 g de solution de silicate de sodium à 27 % en poids de $SiO_2$. 495 g de nitrate de nickel cristallisé sont mis en solution dans 1,4 l d'eau ; on ajoute alors 250 cm³ d'ammoniaque à 13,5 mole/l en $NH_3$ : la solution obtenue est bleu-limpide. Ces 2 solutions sont mélangées ; l'homogénéisation du milieu demande une forte agitation en raison de la densité élevée du silicate de sodium. L'ammoniac est ensuite évaporé lentement sous agitation à 70-80 °C : le précipité obtenu est filtré et lavé avec 5,5 l d'eau déminéralisée. Après séchage à 80 °C pendant 15 h, il est décomposé à 400 °C sous air pendant 5 h puis mis en forme (pastilles cylindriques 5 × 5 mm) ; la montée en température est de 50 °C/h. Il est ensuite réduit à 450 °C pendant 10 h sous mélange $N_2$ + 3 $H_2$ (400 Nl/h) en montant progressivement la température de l'ambiante à 450 °C à raison de 50 °C/h. La teneur en Ni du catalyseur est de 54,4 %, celle en $Al_2O_3$ de 18,4 %, celle en $SiO_2$ de 27,2 % et sa surface spécifique est de 239 m²/g.

Application : Dans l'autoclave, on charge 50 g d'éthanolamine, 140 g d'ammoniac, 10 g d'eau, 7,50 g du catalyseur 11 et 11,8 Nl d'hydrogène. On maintient en palier à 195 °C pendant 8 h, sous agitation, la pression est de 141 bars absolus. Les résultats sont regroupés dans les tableaux ci-après.

| Taux de transformation de E.A. | % |
|---|---|
| en E.D.A. | 29,4 |
| en PIP | 18,5 |
| en lourds | 13,6 |
| TOTAL | 61,5 |

| Rendements % pds/E.A. entrée | |
|---|---|
| en E.D.A. | 28,9 |
| en PIP | 13,1 |
| en lourds | 10,1 |
| en $H_2O$ | 38,0 |
| en E.A. non transformée | 38,5 |
| TOTAL | 128,6 |

| Rendements % pds/E.A. consommée | |
|---|---|
| en E.D.A. | 47,0 |
| en PIP | 21,3 |
| en lourds | 16,4 |

### Exemple 12 (Exemple comparatif)

Catalyseur 12 : (Ni sur alumine) ; 100 g de billes d'alumine (surface spécifique 350 m²/g, diamètre moyen des pores 60 Å) tamisées à 2-5 mm et préalablement séchées à 120 °C pendant 8 h sont traitées par 80 cm³ de soude à 150 g/l en NaOH pendant 1/2 h. Après séparation sur filtre de la solution de soude non imprégnée, elles sont de nouveau séchées à 120 °C pendant 4 h puis imprégnées pendant 2 h par du nitrate de nickel fondu obtenu par fusion à 80 °C de 140 g de nitrate de nickel cristallisé. La température de 80 °C est maintenue durant toute l'imprégnation afin d'éviter la cristallisation du nitrate de nickel. Les billes sont ensuite séchées à 80 °C pendant 5 h et traitées sous air à 400 °C ; ce traitement provoque le dégagement de vapeurs nitreuses : afin d'éviter une réaction trop violente la température est montée lentement à partir de 125 °C, à raison de 25 °C/h, jusqu'à 250 °C, le palier à 400 °C est maintenu pendant 4 h. Elles sont alors réduites sous mélange $N_2 + 3 H_2$ (200 Nl/h) à 450 °C pendant 8 h (montée en température 50 °C/h). La teneur en Ni du catalyseur est de 16,7 %, celle en Na de 2,2 %.

Application : Dans l'autoclave, on charge 50 g d'éthanolamine, 140 g d'ammoniac, 10 g d'eau, 8,4 g du catalyseur 12 et 11,8 Ni d'hydrogène. On maintient en palier à 195 °C pendant 8 h, sous agitation, la pression est de 159 bars absolus. Les résultats sont regroupés dans les tableaux ci-après.

| Taux de transformation de E.A. | % |
|---|---|
| en E.D.A. | 17,4 |
| en PIP | 0,6 |
| en lourds | 1,6 |
| TOTAL | 19,6 |

| Rendements % pds/E.A. entrée | |
|---|---|
| en E.D.A. | 17,1 |
| en PIP | 0,4 |
| en lourds | 1,2 |
| en $H_2O$ | 25,8 |
| en E.A. non transformée | 80,4 |
| TOTAL | 124,9 |

| Rendements % pds/E.A. consommée | |
|---|---|
| en E.D.A. | 87,2 |
| en PIP | 2,2 |
| en lourds | 6,2 |

### Exemple 13

Catalyseur 13 : (Ni sur alumine). Dans 2,8 l d'eau sont mis en solution : 495,4 g de nitrate de nickel cristallisé et 735,5 g de nitrate d'aluminium cristallisé. On ajoute sous agitation 2,8 l d'une solution de soude contenant 372 g de NaOH. Le précipité obtenu est filtré puis lavé avec 6 l d'eau. Il est ensuite séché à 90 °C pendant 12 h et traité à 300 °C sous air pendant 5 h (montée en température de l'ambiante jusqu'à 300 °C à raison de 50 °C/h). Le produit obtenu après refroidissement est broyé finement, mélangé avec 1 % de graphite puis pastillé (pastilles de 5 × 5 mm) ; celles-ci sont réduites sous mélange $Ni + 3 H_2$ (400 Nl/h) ; le gaz réducteur est introduit à froid, la température est montée jusqu'à 450 °C à raison de 50 °C/h et on maintient en palier à 450 °C pendant 8 h. La teneur en Ni du catalyseur est de 50 %.

D'après ce même mode de fabrication, on prépare 5 autres catalyseurs à différentes teneurs en Ni

Catalyseur 14 :  10 % de Ni
Catalyseur 15 :  30 % de Ni
Catalyseur 16 :  44 % de Ni
Catalyseur 17 :  70 % de Ni
Catalyseur 18 :  85 % de Ni

**0 002 630**

La surface spécifique des catalyseurs 13 à 18 est voisine de 100 m$^2$/g.

Application : Dans l'autoclave, on charge 50 g d'éthanolamine, 140 g d'ammoniac, 10 g d'eau, 7,5 g d'un des catalyseurs 13 à 18, et 11,8 Nl d'hydrogène. On maintient en palier à 195 °C pendant 8 h, sous agitation ; la pression est de 146 bars absolus. Les résultats sont regroupés dans le tableau ci-après.

| Taux de trans-formation de E.A. % | Catalyseur 14 | Catalyseur 15 | Catalyseur 16 | Catalyseur 13 | Catalyseur 17 | Catalyseur 18 |
|---|---|---|---|---|---|---|
| en E.D.A. | 8,1 | 18,0 | 27,7 | 31,6 | 32,8 | 34,4 |
| en PIP | 0,4 | 1,8 | 8,1 | 10,3 | 13,4 | 21,6 |
| en lourds | 0 | 1,6 | 5,5 | 6,2 | 8,8 | 12,8 |
| TOTAL | 8,5 | 21,4 | 41,3 | 48,1 | 55,0 | 68,8 |

Exemple 14

Dans un réacteur ouvert vertical, on charge 150 cm$^3$ du catalyseur 4 en pastilles 5 × 5 mm ; la réduction du catalyseur est effectuée in situ ; le débit de gaz réducteur (N$_2$ + 3 H$_2$) est de 800 Nl/h ; la montée de la température est progressive à partir de l'ambiante à raison de 50 °C/h et le palier à 450 °C est maintenu pendant 3 h. Le réacteur est alimenté en continu de haut en bas avec : 72,2 g/h d'éthanolamine, 500 g/h d'ammoniac, 40 Nl/h d'hydrogène, préchauffés à 215 °C ; on maintient une pression de 200 bars absolus et la température du lit catalytique est de 215 °C. La vitesse volumétrique horaire (rapport du débit total du gaz en Nl/h au volume de catalyseur en 1) est de 4 855 h$^{-1}$ ; le temps de contact de l'éthanolamine avec le catalyseur est de 83 s.

Les résultats obtenus, après 4 212 h de fonctionnement sont regroupés dans les tableaux ci-après.

| Taux de transformation de E.A. % | |
|---|---|
| en E.D.A. | 31,9 |
| en PIP | 11,7 |
| en lourds | 9,3 |
| TOTAL | 52,9 |

| Rendements % pds/E.A. entrée | |
|---|---|
| en E.D.A. | 31,4 |
| en PIP | 8,2 |
| en lourds | 6,8 |
| en H$_2$O | 15,3 |
| en E.A. non transformée | 47,2 |
| TOTAL | 108,9 |

| Rendements % pds/E.A. consommée | |
|---|---|
| en E.D.A. | 59,4 |
| en PIP | 15,6 |
| en lourds | 12,9 |

Exemple 15

Dans le réacteur ouvert vertical, on charge 150 cm$^3$ du catalyseur 5 en pastilles 5 × 5 mm ; la réduction du catalyseur est effectuée in situ ; le débit de gaz réducteur (N$_2$ + 3 H$_2$) est de 800 Nl/h ; le palier à 450 °C est maintenu pendant 3 h. Le réacteur est alimenté en continu de haut en bas avec : 72,6 g/h d'éthanolamine, 481 g/h d'ammoniac, 46,4 Nl/h d'hydrogène, préchauffés à 220 °C ; on maintient une pression de 200 bars absolus et la température du lit catalytique est de 220 °C. La vitesse volumétrique horaire est de 4 750 h$^{-1}$ ; le temps de contact de l'éthanolamine avec le catalyseur est de 84 s. Les résultats obtenus, après 4 508 h de fonctionnement, sont regroupés dans les tableaux ci-après.

(Voir Tableaux p. 11)

10

| Taux de transformation de E.A. | % |
|---|---|
| en E.D.A. | 36,8 |
| en PIP | 8,5 |
| en lourds | 8,7 |
| TOTAL | 54,0 |

| Rendements % pds/E.A. entrée | |
|---|---|
| en E.D.A. | 36,2 |
| en PIP | 6,0 |
| en lourds | 6,3 |
| en H$_2$O | 15,7 |
| en E.A. non transformée | 46,0 |
| TOTAL | 110,2 |

| Rendements % pds/E.A. consommée | |
|---|---|
| en E.D.A. | 67,1 |
| en PIP | 11,1 |
| en lourds | 11,7 |

## Exemple 16

Dans le réacteur ouvert vertical, chargé avec le catalyseur 4 de l'exemple 14, on pratique 2 séries d'essais avec ou sans recyclage de pipérazine.

Les conditions des essais et les résultats obtenus sont regroupés dans les tableaux ci-après.

| Conditions | | Cas 1 | Cas 2 | Cas 3 | Cas 4 |
|---|---|---|---|---|---|
| Débits entrée | E.A. g/h | 72,0 | 67,7 | 71,7 | 66,5 |
| | PIP g/h | 0 | 4,3 | 0 | 5,2 |
| | NH$_3$ g/h | 505 | | 500 | |
| | H$_2$ Nl/h | 41,0 | | 43,8 | |
| Température catalyseur °C | | 210 | | 215 | |

| | | |
|---|---|---|
| Pression bars absolus | 200 | 170 |
| Vitesse volumétrique horaire h$^{-1}$ | 4900 | 4850 |
| Temps de contact E.A. ou mélange (EA., PIP) | 82 | 72 |

Résultats cas 1 et 2

| Taux de transformation de E.A. % | Cas 1 | Cas 2 |
|---|---|---|
| en E.D.A. | 27,9 | 30,9 |
| en PIP | 10,1 | 4,5 |
| en lourds | 9,3 | 9,7 |
| TOTAL | 47,3 | 45,1 |

| Rendements % pds/E.A. entrée | Cas 1 | Cas2 |
|---|---|---|
| en E.D.A. | 27,4 | 30,4 |
| en PIP | 7,1 | 3,2 |
| en lourds | 6,8 | 7,1 |
| en H$_2$O | 13,6 | 13,0 |
| en E.A. non transformée | 52,8 | 54,9 |
| TOTAL | 107,7 | 108,6 |

11

| Rendements % pds/E.A. consommée | Cas 1 | Cas 2 |
|---|---|---|
| en E.D.A. | 58,0 | 67,4 |
| en PIP | 15,1 | 7,0 |
| en lourds | 14,4 | 15,8 |

Résultats cas 3 et 4

| Taux de transformation de E.A.          % | Cas 3 | Cas 4 |
|---|---|---|
| en E.D.A. | 23,9 | 25,8 |
| en PIP | 6,5 | 1,6 |
| en lourds | 7,8 | 9,7 |
| TOTAL | 38,2 | 37,1 |

| Rendements % pds/E.A. entrée | Cas 3 | Cas 4 |
|---|---|---|
| en E.D.A. | 23,5 | 25,4 |
| en PIP | 4,6 | 1,1 |
| en lourds | 5,7 | 7,0 |
| en $H_2O$ | 11,0 | 10,6 |
| en E.A. non transformée | 61,9 | 63,0 |
| TOTAL | 106,7 | 107,1 |

| Rendements % pds/E.A. consommée | Cas 3 | Cas 4 |
|---|---|---|
| en E.D.A. | 61,7 | 68,5 |
| en PIP | 12,0 | 3,1 |
| en lourds | 14,8 | 18.9 |

Exemple 17

Exemple catalyseur 19 (Nickel sur alumine). 735 g de nitrate d'aluminium cristallisé $Al(NO_3)_3$, $9H_2O$ et 389 g de nitrate de nickel cristallisé $Ni(NO_3)_2$, $6H_2O$ sont mis en solution dans 1 l d'eau. A cette solution est ajoutée sous agitation 800 $cm^3$ d'une solution de soude contenant 342 g de NaOH.

La suspension est ensuite portée à l'ébullition pendant 20 mn ; après refroidissement à 30 °C, le précipité est filtré sur verre fritté puis lavé avec environ 400 $cm^3$ d'eau à 20 °C ; le volume d'eau utilisé est ensuite séché à 80 °C puis décomposé à 350 °C pendant 6 h. Après mélange avec 2 % de graphite, la poudre est mise en forme (pastilles cylindriques 5 × 5 mm).

Dans un réacteur ouvert vertical, on charge 130 $cm^3$ de ce catalyseur, la réduction du catalyseur est effectuée in situ, sous mélange de gaz réducteur à 450 °C pendant 8 h ; la montée en température étant de 50 °C/h.

Les teneurs en Ni, Na du catalyseur réduit sont respectivement de 39,2 %, 8 %. La surface spécifique est de 43 $m^3$ g. La résistance mécanique à l'écrasement du catalyseur est de 50 kg sur les faces plates.

Application

Le réacteur est alimenté en continu de haut en bas avec : 69,2 g/h d'éthanolamine, 249 g/h d'ammoniac, 215 Nl/h d'hydrogène, préchauffés à 215 °C ; on maintient une pression de 185 bars absolus et la température du lit catalytique est de 215 °C. La vitesse volumétrique horaire est de 5 600 $h^{-1}$ ; le temps de contact de l'éthanolamine avec le catalyseur est de 67 s.

Les résultats obtenus, après 5 030 h de fonctionnement, sont regroupés dans les tableaux ci-après et sont pratiquement identiques à ceux du début de la mise en opération.

Conclusion

L'activité est restée parfaitement stable et l'examen du catalyseur montre qu'il n'a subi aucune altération, la résistance mécanique, après 5 030 h, étant de 55 kg sur les faces plates.

| Taux de transformation de E.A. % |  |
|---|---|
| en E.D.A. | 31,4 |
| en PIP | 6,6 |
| en lourds | 5,7 |
| TOTAL | 43,7 |

| Rendements % pds/E.A. entrée |  |
|---|---|
| en E.D.A. | 30,9 |
| en PIP | 4,6 |
| en lourds | 4,4 |
| en H$_2$O | 12,9 |
| en E.A. non transformée | 56,3 |
| TOTAL | 109,1 |

| Rendements % pds/E.A. consommée |  |
|---|---|
| en E.D.A. | 70,7 |
| en PIP | 10,6 |
| en lourds | 10,1 |

## Exemple 18

Catalyseur 20 (Nickel, Sodium, Rhodium sur alumine). A 286,5 Cm$^3$ d'une solution de nitrate de nickel de densité 1,53 contenant 14,26 % en poids de Ni, on ajoute 1 200 cm$^3$ d'une solution de nitrate d'aluminium contenant 459,7 g en nitrate Al (NO$_3$)$_3$, 9 H$_2$O. La solution obtenue est ajoutée sous agitation dans une solution de soude à 100 g/l contenant 232,5 g en NaOH. La suspension est portée à l'ébullition pendant 10 mn ; après refroidissement, elle est filtrée sur un entonnoir filtrant et lavée avec 300 cm$^3$ d'eau à 20 °C. Le seuil d'arrêt du lavage est déterminé afin d'obtenir une teneur en sels de sodium au niveau indiqué ci-après. Le précipité est séché à 100 °C puis décomposé à 350 °C en montant la température progressivement à raison de 50 °C/h et en maintenant le palier à 350 °C pendant 6 h. Le produit est broyé finement puis imprégné avec 55 cm$^3$ de solution de sel de rhodium (aquo pentachlorure d'ammonium) (NH$_4$)$_2$Rh H$_2$O Cl$_5$ contenant 71,6 mg de Rh. Après séchage à 80 °C, le produit est mélangé avec 2 % de graphite, puis mis en forme (pastilles cylindriques 5 × 5 mm).

Dans un réacteur ouvert vertical on charge 130 cm$^3$ de ce catalyseur la réduction du catalyseur est effectuée in situ, sous mélange de gaz réducteur à 450 °C pendant 8 h.

Les teneurs en Ni, Rh, Na du catalyseur réduit sont respectivement de 43,6 %, 0,05 %, 9,5 %. La surface spécifique est de 37 m$^2$/g. La résistance mécanique à l'écrasement du catalyseur est de 62 kg sur les faces plates.

Application

Le réacteur est alimenté en continu de haut en bas avec : 69,4 g/h d'éthanolamine, 493 g/h d'ammoniac, 70 Nl/h d'hydrogène, préchauffés à 215 °C ; on maintient une pression de 190 bars absolus et la température du lit catalytique est de 215 °C. La vitesse volumétrique horaire est de 5 800 h$^{-1}$, le temps de contact de l'éthanolamine avec le catalyseur est de 66 s.

Les résultats obtenus, après 4 823 h de fonctionnement, sont regroupés dans les tableaux ci-après et sont pratiquement identiques à ceux du début de l'opération.

| Taux de transformation de E.A. % |  |
|---|---|
| en E.D.A. | 32,2 |
| en PIP | 7,3 |
| en lourds | 6,3 |
| TOTAL | 45,8 |

| Rendements % pds/E.A. entrée |  |
|---|---|
| en E.D.A. | 31,7 |
| en PIP | 5,1 |
| en lourds | 4,8 |
| en H$_2$O | 13,4 |
| en E.A. non transformée | 54,2 |
| TOTAL | 109,2 |

| Rendements % pds/E.A. consommée |  |
|---|---|
| en E.D.A. | 69,2 |
| en PIP | 11,2 |
| en lourds | 10,4 |

Conclusion

L'activité est plus élevée en raison de la présence de rhodium. L'activité est demeurée parfaitement stable. Le catalyseur n'a subi aucune altération, la résistance mécanique est conservée, on trouve 70 kg.

# 0 002 630

**Revendications**

1. Catalyseur d'amination en vue de la production d'éthylène diamine et de pipérazine à partir de l'éthanolamine, dont le métal actif est de la famille des métaux de transition dont la teneur représente 30 à 70 % du poids du catalyseur, l'association du métal de transition et du support microporeux en édifice structural étant obtenue par co-précipitation de tous les éléments du catalyseur à partir de sels solubles ou par imprégnation d'un sel soluble des éléments actifs sur les oxydes réfractaires préalablement combinés, suivie d'un traitement thermique et d'une réduction, caractérisé en ce que le métal actif est le nickel seul ou en association avec d'autres métaux de transition tels cuivre ou cobalt ; le nickel représentant au moins 50 % de la matière active, uniformément allié à un édifice poreux réfractaire de surface spécifique comprise entre 10 et 300 m²/g et de diamètre de pores inférieur à 5 000 Å, choisi parmi le groupe constitué par l'alumine, la silice, la thorine et l'oxyde de cérium et en ce que l'association du métal de transition et du support microporeux est soumise à un traitement thermique à l'air à une température comprise entre 350 et 500 °C, pendant quelques heures, avec une montée progressive en température depuis l'ambiante de 50 °C par heure, puis à une dite réduction directe sous balayage de gaz réducteur entre 400 et 550 °C pendant plusieurs heures, avec une montée en température de l'ambiante à la température de réduction de 50 °C par heure.

2. Catalyseur d'amination selon la revendication 1, caractérisé en ce que la surface spécifique est comprise entre 30 et 50 m²/g.

3. Catalyseur d'amination selon la revendication 1, caractérisé en ce que le catalyseur contient un stabilisant constitué par un composé à base de sodium introduit à une teneur comprise entre 0,15 et 20 % exprimée en sodium par rapport au poids du catalyseur.

4. Catalyseur d'amination selon la revendication 5, caractérisé en ce que la teneur en sodium est comprise entre 5 et 10 % en poids.

5. Catalyseur d'amination selon la revendication 1, caractérisé en ce que le catalyseur contient un promoteur associé au métal actif, le dit promoteur étant un composé à base de rhodium dont la teneur maximale est de 0,1 % exprimée en rhodium par rapport au poids du catalyseur.

6. Application du catalyseur selon la revendication 1, à la réaction catalytique en phase hétérogène, de l'éthanolamine et de l'ammoniac conduite à une température comprise entre 170 et 260 °C, sous une pression comprise entre 50 et 300 bars absolus et selon laquelle l'éthanolamine, l'ammoniac et l'hydrogène sont introduits en quantités telles que le rapport molaire ammoniac/éthanolamine soit compris entre 5 et 40 et le débit d'hydrogène soit compris entre 5 et 200 Nl par mole d'éthanolamine.

7. Application du catalyseur dans le procédé selon la revendication 6, dans lequel la réaction est conduite en présence d'eau introduite dans le mélange réactionnel jusqu'à concurrence de 25 % en poids par rapport à l'éthanolamine.

8. Application du catalyseur dans le procédé selon la revendication 7, caractérisée par le recyclage des réactifs et le recyclage au moins partiel d'un des réactants.

**Claims**

1. Amination catalyst for the production of ethylene diamine and of piperazine from ethanolamine, the active metal of which is from the family of transition metals, the proportion of which represents 30 % to 70 % by weight of the catalyst, the association of transition metal and microporous support into a structural body being obtained by co-precipitation of all the ingredients of the catalyst by means of soluble salts or by impregnation of a soluble salt of the active elements into the previously combined refractory oxides followed by a thermal treatment and reduction, characterized in that the active metal in nickel solely or in association with other transition metals such as copper or cobalt ; the nickel representing at least 50 % of the active material, uniformly combined into a porous refractory structure with a specific surface between 10 and 300 m²/g and with a pore diameter smaller than 5 000 Å, slected from the group consisting of alumina, silica, thoria and cerium oxide, and that the associaiton of transition metal and micro porous support is subjected to a thermal treatment with air at a temperature between 350 and 500 °C, for several hours, with a progressive increase in temperature of 50 °C per hour from the ambient, then to a said direct reduction under a flow of reducing gas between 400 and 550 °C for several hours, with an increase in temperature of 50 °C per hour from the ambient to the temperature of reduction.

2. Amination catalyst according to claim 1, characterized in that the specific surface is between 30 and 50 m²/g.

3. Amination catalyst according to claim 1, characterized in that the catalyst contains a stabilizer constituted by a compound based on sodium introduced at a percentage between 0,15 and 20 %, expressed as sodium, based on the weight of the catalyst.

4. Amination catalyst according to claim 3, characterized in that the content of sodium is between 5 and 10 % by weight.

5. Amination catalyst according to claim 1, characterized in that the catalyst contains a promoter associated with the active metal, the said promoter being a composition based on rhodium, the maximum proportion of which is 0,1 % expressed as rhodium, based on the weight of the catalyst.

6. Use of the catalyst according to claim 1 in the catalytic heterogeneous phase reaction of ethanolamine and ammonia carried out at a temperature between 170 and 260 °C, under a pressure between 50 and 300 bars absolute, the ethanolamine, ammonia and hydrogen being introduced in such quantities that the molar ratio of ammonia at ethanolamine is between 5 and 40 and the consumption of hydrogen is between 5 and 200 Nl per mole of ethanolamine.

7. Use of the catalyst in the process according to claim 6, wherein the reaction is carried out in presence of water introduced into the reaction mixture up to an amount of 25 % by weight based on the ethanolamine.

8. Use of the catalyst in the process according to claim 6, characterized by recycling the reagents and at least partial recycling one of the reactents.


## Patentansprüche

1. Aminierungskatalysator zur Herstellung von Äthylendiamin und Piperazin mit Hilfe von Äthanolamin, dessen aktives Metall aus der Familie der Übergangsmetalle stammt, dessen Gehalt 30 bis 70 Gew.-% des Katalysators ausmacht und wobei die Vereinigung des Übergangsmetalles mit dem mikroporösen Trägerkörper durch gemeinsame Ausfällung aller Katalysatorelemente mit Hilfe von löslichen Salzen oder durch Imprägnierung der vorher miteinander vereinigten hitzebeständigen Oxide mit einem löslichen Salz der aktiven elemente, anschließende Hitzebehandlung und Reduktion erhalten wurde, dadurch gekennzeichnet, daß das aktive Metall Nickel alleine oder in Verbindung mit anderen Übergangsmetallen, wie Kupfer oder Kobalt ist, daß Nickel wenigstens 50 % des aktiven Materials ausmacht und gleichmäßig mit einem porösen hitzebeständigen Körper mit einer spezifischen Oberfläche zwischen 10 und 300 m²/g und mit einem Porendurchmesser kleiner als 5 000 Å aus der Gruppe Aluminiumoxid, Kieselsäure, Thoriumoxid und Ceroxid vereinigt ist und daß die Vereinigung des Übergangsmetalles und des mikroporösen Trägers durch eine Hitzebehandlung mit Luft bei einer Temperatur zwischen 350 und 500 °C während mehrerer Stunden mit einer progressiven Temperatursteigerung von 50 °C je Stunde, ausgehend von Umgebungstemperatur, und durch anschließende direkte Reduktion unter Durchspülung mit dem reduzierenden Gas bei 400 bis 550 °C während mehrerer Stunden mit einer Temperatursteigerung von 50 °C je Stunde, ausgehend von Umgebungstemperatur bis zur Reduktionstemperatur, erhalten wurde.

2. Aminierungskatalysator nach Anspruch 1, dadurch gekennzeichnet, daß die spezifische Oberfläche zwischen 30 und 50 m²/g liegt.

3. Aminierungskatalysator nach Anspruch 1, dadurch gekennzeichnet, daß der Katalysator einen Stabilisator enthält, der aus einer Verbindung auf Natriumgrundlage besteht und mit einem Gehalt von 0,15 bis 20 %, ausgedrückt als Natrium, des Katalysatorgewichtes eingeführt ist.

4. Aminierungskatalysator nach Anspruch 3, dadurch gekennzeichnet, daß der Gehalt an Natrium zwischen 5 und 10 Gew.-% liegt.

5. Aminierungskatalysator nach Anspruch 1, dadurch gekennzeichnet, daß der Katalysator einen mit aktivem Metall verbundenen Promotor enthält, wobei dieser Promotor eine Verbindung auf Rhodiumgrundlage ist, deren Gehalt maximal 0,1 %, ausgedrückt als Rhodium, des Katalysatorgewichtes ist.

6. Verwendung des Katalysators nach Anspruch 1 in heterogener Phase zur katalytischen Umsetzung von Äthynolamin und Ammoniak bei einer Temperatur zwischen 170 und 260 °C unter einem Druck zwischen 50 und 300 bar absolut, wobei das Äthanolamin, das Ammoniak und der Wasserstoff in solchen Mengen eingeführt werden, daß das Molverhältnis von Ammoniak/Äthanolamin zwischen 5 und 40 und der wasserstoffdurchsatz zwischen 5 und 200 Nl je Mol Äthanolamin liegt.

7. Verwendung des Katalysators nach Anspruch 6, wobei die Umsetzung in Gegenwart von Wasser durchgeführt wird, das in das Reaktionsgemisch bis zu einer Menge von 25 Gew.-% in Bezug auf das Äthanolamin eingeführt wird.

8. Verwendung des Katalysators in dem Verfahren nach Anspruch 6, gekennzeichnet durch die Rückführung der Reagenzien und die Rückführung wenigstens eines Teils eines der Reaktionsteilnehmer.